# EUROPÄISCHE PATENTSCHRIFT

(11) **EP 1 025 869 B1**
(45) Veröffentlichungstag und Bekanntmachung des Hinweises auf die Patenterteilung: **07.01.2004**
(21) Anmeldenummer: 00102072.6
(22) Anmeldetag: 02.02.2000
(51) Int. Cl.: A61L 27/00, A61K 9/50, C12N 5/06, C12N 11/04, C08L 5/04

(54) **Verfahren zur Herstellung von stabilem Alginatmaterial**
Process for the manufacture of stable alginate material
Procédé pour la fabrication d'un matériau stable à base d'alginate

(30) Priorität: 05.02.1999 DE 19904785
(43) Veröffentlichungstag der Anmeldung: 09.08.2000
(73) Patentinhaber: CellMed AG, 63755 Alzenau (DE)
(72) Erfinder: Zimmermann, Ulrich, 97295 Waldbrunn (DE)
(74) Vertreter: Schneider, Michael, Dr.

(56) Entgegenhaltungen:
- WO-A-91/09119
- PATENT ABSTRACTS OF JAPAN vol. 13, no. 372 (C-627), 17. August 1989 (1989-08-17) & JP 01 128787 A (KOMATSU ), 22. Mai 1989 (1989-05-22) & DATABASE WPI Week 198926 Derwent Publications Ltd., London, GB; AN 189420
- DRAGET K I ET AL: "Swelling and partial solubilization of alginic acid gel beads in acidic buffer" CARBOHYDRATE POLYMERS,GB,APPLIED SCIENCE PUBLISHERSLTD. BARKING, Bd. 29, Nr. 3, 1. März 1996 (1996-03-01), Seiten 209-215, XP004069606 ISSN: 0144-8617

## Beschreibung

Die Erfindung betrifft die Herstellung von polymerem Alginatmaterial, insbesondere die Herstellung von Alginatkapseln oder - schichten, Verwendungen derartiger Alginatmaterialien und biologische Implantate, die mit derartigen Alginatmaterialien umhüllt oder beschichtet sind.

Es ist bekannt, bei allogenen oder xenogenen Transplantationen Immunreaktionen des Wirtsorganismus durch eine Mikroverkapselung des Transplantats zu dessen Immunisolation zu begegnen (s. F. Lim et al. in "Science", Bd. 210, 1980, S. 908-910, H. A. Clayton et al. in "Acta Diabetol.", Bd. 30, 1993, S. 181-189). Für die Verkapselung allogenen oder xenogenen, endokrinen Gewebes haben sich sphärische Alginatkapseln als vorteilhaft erwiesen (s. z.B. T. Zekorn et al. in "Acta Diabetol.", Bd. 29, 1992, S. 41-52, P. De Vos et al. in "Biomaterials", Bd. 18, 1997, S. 273-278, C. Hasse et al. in "Exp. Clin. Endocrinol. Diabetes", Bd. 105, 1997, S. 53-56 und in "J. Microencapsulation", Bd. 14, 1997, S. 617-626). Die Alginate sind Ca²⁺- oder Ba²⁺-vernetzt und für die Immunisolation besonders wirksam, wenn sie gereinigt, d.h. möglichst frei von mitogenen Kontaminationen sind (s. U. Zimmermann et al. in "Electrophoresis", Bd. 13, 1992, S. 269-274, G. Klöck et al. in "Biomaterials", Bd. 18, 1997, S. 707-713).

Die Transplantation alginatumhüllten Gewebes wird nicht nur in Tierversuchen getestet, sondern auch schon in die Humanmedizin eingeführt. So wird beispielsweise von C. Hasse et al. in "The Lancet", Bd. 350, 1997, S. 1296, die Mikroverkapselungstechnik bei einer allogenen Nebenschilddrüsentransplantation im Muskelgewebe zweier Patienten mit permanenter symptomatischer Unterfunktion der Nebenschilddrüsen beschrieben. Nach der Behandlung mit Alginat-immunisoliertem Nebenschilddrüsengewebe, konnten die Patienten aus dem Krankenhaus entlassen werden, ohne hypokalzämische Symptome zu zeigen und ohne eine Therapie zur Unterdrückung von Immunreaktionen zu benötigen. Allerdings zeigte sich, wie schon bei Tierversuchen, daß die Transplantate nur für eine beschränkte Zeit wirksam waren, was auf das Verschwinden der Alginatumhüllungen zurückzuführen ist.

Aus US 5 429 821 ist die Bildung alginatbeschichteter Transplantate bekannt, bei denen zur Erhöhung der Alginatstabilität Ca²⁺-vernetztes Alginatgel mit L-poly-Lysin oder anderen physiologisch annehmbaren nicht-giftigen Polykationen vernetzt wird. Diese Technik besitzt zwar den Vorteil einer erhöhten Alginatstabilität. Die Vernetzung mit L-poly-Lysin ist jedoch nachteilig, da damit durch das eingepflanzte Transplantat eine Fibrose ausgelöst werden kann, so daß sich Probleme wie bei der Verwendung nicht-gereinigter Alginate ergeben. Es ist daher eine weitere Behandlung der Alginatgele zur Erzielung einer Biokompatibilität des Transplantats erforderlich.

Die Stabilität der transplantierten Alginatkapseln ist ein generelles Problem, das beispielsweise auch von G. Klöck et al. in "Biomaterials", Bd. 18, 1997, S. 707-713, und P. De Vos in "Diabetologia", Bd. 40, 1997, S. 262-270, beschrieben wird. Der weitere klinische Einsatz der alginatumhüllten Transplantate setzt die Entwicklung stabilerer Alginatkapseln voraus.

Die Aufgabe der Erfindung besteht darin, ein Verfahren zur Herstellung von Alginatmaterial, insbesondere zur Umhüllung oder Beschichtung von Transplantaten, anzugeben, daß eine erhöhte Stabilität und Zuverlässigkeit des Alginatmaterials unter Einsatzbedingungen liefert. Die Aufgabe der Erfindung besteht auch darin, neue Verwendungen derartigen Alginatmaterials und neue Transplantate anzugeben, die mit einem derart verbesserten Alginatmaterial versehen sind.

Diese Aufgaben werden durch ein Verfahren und durch Transplantatkomposite mit den Merkmalen gemäß den Patentansprüchen 1 bzw. 12 gelöst. Vorteilhafte Ausführungsformen und Verwendungen der Erfindung ergeben sich aus den abhängigen Ansprüchen.

Die Erfindung basiert auf dem Gedanken, nach der Bildung eines vernetzten Alginatmaterials durch Ausfällung gelösten Alginats in einer Fällungslösung das Alginatmaterial einer Behandlungslösung auszusetzen, in der der Unterschied des chemischen Potentials wäßriger Bestandteile im Alginatmaterial und des Wassers in der Behandlungslösung oder der kolloidosmotische Druck zwischen dem Alginatmaterial und der Behandlungslösung minimiert oder zum Wert Null ausgeglichen wird. Hierzu wird vorzugsweise die Lösung einer Substanz, die überschüssige zweiwertige (divalente) Kationen im Alginat bindet, wie eine Sulfatlösung (z.B. Na₂SO₄-Lösung) als Behandlungslösung verwendet, um die überschüssigen Ionen im Alginatmaterial zu binden und simultan den genannten Unterschied der thermodynamischen Potentiale bzw. den kolloidosmotischen Druck zu verringern.

Die erfindungsgemäße Behandlung des vernetzten Alginatmaterials wird vorzugsweise bei der Herstellung von Alginatkapseln zur Umhüllung von Transplantatmaterialien oder Wirkstoffen verwendet, die in den menschlichen Organismus eingebracht werden sollen. Erfindungsgemäß hergestellte Alginatkapseln zeigen im Vergleich zu herkömmlichen Alginatkapseln eine wesentlich erhöhte Stabilität.

Gemäß bevorzugter Ausführungsformen der Erfindung erfolgt die Bildung des vernetzten Gelmaterials aus einer modifizierten Alginatlösung. Hierzu werden der Alginatlösung biologisch verträgliche Puffersubstanzen, Proteine und/oder hydrophobe Substanzen zugesetzt.

Gegenstand der Erfindung ist auch ein Transplantatkomposit, bestehend aus einem Kompositkern mit einer Alginatumhüllung, wobei der Kompositkern biologische Zellen und/oder dispergierte Wirkstoffe umfaßt und die Alginathülle nach dem erfindungsgemäßen Verfahren hergestellt ist. Die biologischen Zellen sind beispielsweise Hormon- oder Antikörper-produzierende, lebende Zellen. Die verkapselten Wirkstoffe umfassen beispielsweise medizinisch oder ernährungsphysiologisch wirksame Substanzen oder auch Markierungssubstanzen, die dazu vorgesehen sind, nach Einbringung in den menschlichen Organismus durch ein äußeres Meßverfahren in Bezug auf vorbestimmte Stoffeigenschaften in Abhängigkeit von der jeweiligen Umgebung erfaßt zu werden.

Die Erfindung besitzt die folgenden Vorteile. Es werden erstmalig Alginatumhüllungen geschaffen, die eine erheblich höhere Stabilität als herkömmliche Alginatumhüllungen besitzen. Damit wird insbesondere erstmalig die Möglichkeit geschaffen, reproduzierbare Langzeituntersuchungen zur Transplantation alginatumhüllter Stoffe in tierischen Organismen oder an Patienten durchzuführen.

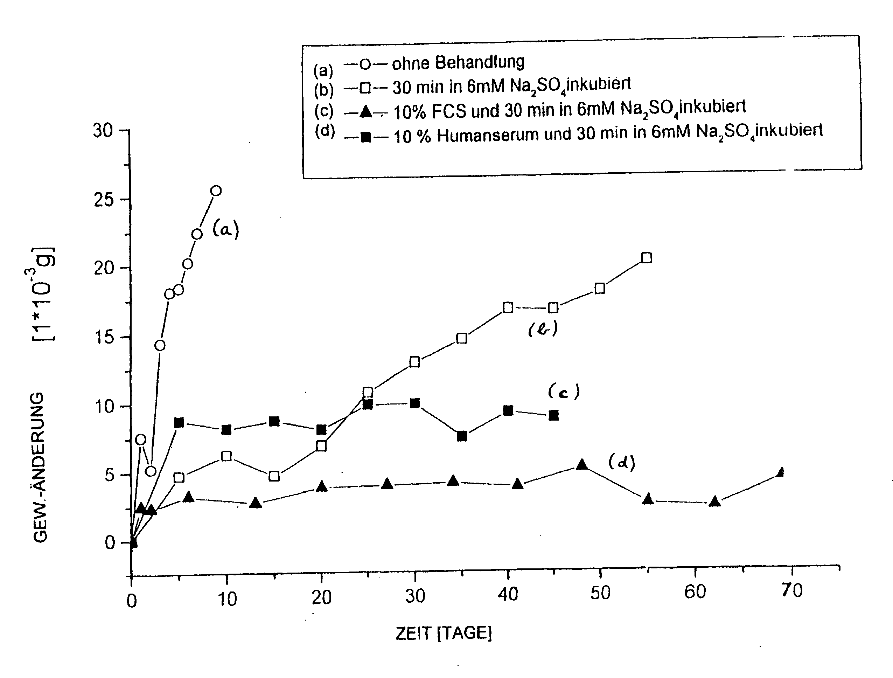

Weitere Einzelheiten und Vorteile der Erfindung werden aus der folgenden Beschreibung unter Bezugnahme auf die beigefügte Figur ersichtlich. Fig. 1 zeigt eine Kurvendarstellung des Gewichts von Alginatkapseln in Abhängigkeit von der Zeit zur Illustration der Wirkung der erfindungsgemäßen Behandlung vernetzten Alginatmaterials.

Die Erfindung wird im folgenden unter Bezug auf die Herstellung von kapselförmgien Alginatumhüllungen für mikroskopisch kleine Partikel beschrieben, die in einen lebenden Organismus eingebracht werden sollen. Die Erfindung ist auf diese Anwendung jedoch nicht beschränkt, sondern bei der Herstellung von Alginatmaterial auch in anderen Formen (z.B. Trägerschichten für Zellkulturen, ebene Umhüllungsschichten, Zwischenschichten in Transplantatkompositen, Material in Wundverbänden, Material in sog. "functional food" u. dgl.) anwendbar.

Die Bildung vernetzten Alginatmaterials in Form von mikroskopisch kleinen Kapseln erfolgt beispielsweise entsprechend der Verfahrensweisen, die von P. Gröhn et al. in "Exp. Clin. Endocrinal.", Bd. 102, 1994, S. 380 ff., oder von C. Hasse et al. in "The Lancet", Bd. 350, 1997, S. 1296, beschrieben sind. Die dort beschriebenen Einzelheiten zur Isolierung des Transplantatmaterials, zur Alginatvorverarbeitung, zur Verkapselung des Transplantatmaterials und zur Reinigung des verkapselten Transplantatmaterials werden hiermit vollständig in die vorliegende Beschreibung einbezogen. Die Herstellung der Alginatkapseln (sogenannte "Beads") erfolgt vorzugsweise durch Ausfällung einer Alginatlösung in einer zwei- oder mehrwertigen, ionenenthaltenden Fällungslösung unter Verwendung der in der genannten Publikation beschriebenen Zwei- oder Drei-Kanal-Tropfentechnik.

Die Alginatkapseln, die im folgenden unter Bezug auf die Meßergebnisse in Fig. 1 beschrieben werden, wurden unter den folgenden Bedingungen hergestellt. Hochgereinigtes Alginat wurde in einer 9%-igen NaCl-Lösung aufgelöst. Die viskose Lösung wurde mit einer konstanten Rate von rd. 0.2 ml/min durch den inneren Kanal eines Zwei-Kanal-Generators gedrückt. Die Größe und dementsprechend das Gewicht der Kapseln wurde durch Zufuhr eines koaxialen Luftstromes gesteuert. Die Tropfen der Alginatlösung fielen in eine iso-osmolare Ba²⁺-Lösung (20 mM) mit 0.72% NaCl. Nach ungefähr 1 Minute wurden die Kapseln entfernt und für die Vergleichsprobe (Kurve (a)) dreimal mit 0.9%-iger NaCl-Lösung gewaschen bzw. bei den erfindungsgemäßen Proben für rd. 30 Minuten in einer isoosmolaren NaCl-Lösung mit Na₂SO₄ (6 mM) inkubiert (Kurven (b) bis (d)). Die Größe und dementsprechend das Gewicht der Kapseln wurden standardmäßig mit einem Mikroskop (Zeiss) und einer Präzisionswaage (Sartorius) gemessen. Im Falle der erfindungsgemäß behandelten Proben (Kurven (c) und (d)) wurde diese Verfahrensweise durch Zusatz von menschlichem Albumin (c) bzw. 10%-igem fetalen Kälberserum (d) zur Alginat- und Fällungslösung modifiziert. Diese Zusätze können auch der Umgebungslösung bei der weiteren Handhabung der Kapseln zugefügt sein.

Die Erfindung basiert auf den folgenden Feststellungen. Werden Alginatkapseln nach ihrer Bildung in einer Fällungslösung (z.B. mit 20 mM Ca²⁺- oder Ba²⁺-Ionen) in eine isoosmolare NaCl-Lösung (T = 37°C) überführt, ohne daß ein zusätzlicher Behandlungsschritt des vernetzten Alginatmaterials gemäß der Erfindung ausgeführt wird, so ergibt sich das in Fig. 1 mit Kreisen gezeichnete Kurvenbild (a). Die Alginatkapseln schwellen an, was sich in der dargestellten Massezunahme im Zeitverlauf äußert. Nach ungefähr 1 Woche erfolgt ein Durchbruch oder Platzen der Kapselmaterials und die Kapseln lösen sich auf. Es wurde festgestellt, daß dieses Phänomen des Anschwellens der Alginatkapseln, das schließlich zu deren Auflösung führt, von der Anwesenheit von zweiwertigen Überschuß-Kationen abhängt, die nach dem Polymerisations- oder Vernetzungsprozeß in der wässrigen Phase der vernetzten Alginat-Gelmatrix zurückbleiben und einen Wassertransport aus der Umgebung in das Matrixmaterial verursachen. Die zweiwertigen Kationen sind ursprünglich in der Fällungslösung enthalten und dienen bei der Bildung des vernetzten Alginatmaterials zur Absättigung freibleibender, negativ geladener Carboxylgruppen in der Gelmatrix. Die freibleibenden Carboxylgruppen sind jene Carboxylgruppen der Gulloronsäure- und Manuronsäure-Ketten des Alginats, die nicht an der Vernetzung teilnehmen. Die Zahl der nach der Vernetzung verbleibenden Überschußkationen und somit das Auftreten des oben beschriebenen Auflösungsphänomens hängt somit von der Monomerzusammensetzung, der Blockstruktur und dem Molekulargewicht der Alginatmoleküle ab, die als Ausgangsmaterial verwendet werden.

Es wurde ferner festgestellt, daß ein kolloidosmotischer Druck durch organische Puffermoleküle (wie z.B. MOPS, HEPES) gebildet wird, die mit typischen Konzentrationen von beispielsweise 10 mM der Fällungslösung zugesetzt sind und während des Vernetzungsvorgangs in die Gelmatrix eingebaut werden und, wie die Carboxylgruppen, sich mit den zweiwertigen Kationen verbinden. Diese Bindungen sind durch verhältnismäßig hohe elektrostatische Kräfte gekennzeichnet, so daß die zweiwertigen Ionen kaum oder nur sehr langsam durch einwertige Ionen ausgewaschen werden können. Die zweiwertigen Überschuß-Kationen verursachen jedoch den kolloidosmotischen Druck, der zu einer Wasseraufnahme führt, die das genannte Anschwellen der Kapseln auslöst.

Zur Vermeidung der beschriebenen Wasseraufnahme des vernetzten Alginatmaterials wird erfindungsgemäß eine Behandlung des Alginatmaterials mit einer Behandlungslösung vorgeschlagen, die dazu vorgesehen ist, einen Gleichgewichtszustand zwischen den wässrigen Restlösungsteilen in der Gelmatrix und der Umgebungslösung möglichst schnell einzustellen. Die Behandlungslösung enthält vorzugsweise zwei- oder mehrwertige Anionen, um die Überschuß-Kationen in der Gelmatrix möglichst schnell zu binden. Die Behandlungslösung ist vorzugsweise eine Sulfatlösung, z.B. eine Na₂SO₄-Lösung, oder eine komplexierende oder fällende Lösung.

Die erfindungsgemäße Behandlung des vernetzten Alginatmaterials mit der Sulfatlösung wird für eine anwendungsabhängig vorbestimmte Zeit vorgenommen. Typische Werte für die Inkubationszeit liegen bei einer Na₂SO₄-Lösung mit Konzentrationen im Bereich von 0.4 bis 20 mM in Zeitbereichen von 10 bis 60 Minuten. Die mit Quadraten gezeichnete Kurve (b) in Fig. 1 zeigt eine Verzögerung des Anschwellens der Beads auf einen Zeitbereich von rd. 50 Tagen bei einer 30-minütigen Behandlung des vernetzten Alginats in einer 6 mM-Na₂SO₄-Lösung. Die Behandlung des Alginatmaterials erfolgt vorzugsweise bei Raumtemperatur oder physiologisch interessierenden Temperaturen (T = 37°C).

Das Alginatmaterial wird unmittelbar nach der Vernetzung der Behandlungslösung ausgesetzt. Es kann aber auch vorgesehen sein, falls das Alginatmaterial unmittelbar nach der Vernetzung zunächst getrocknet wird, daß die Behandlungslösung erst später beim Einsatz des Alginatmaterials zugesetzt wird. Gegenstand der Erfindung ist somit allgemein auch die Behandlung vernetzten Alginatmaterials mit einer Sulfatlösung zur Durchführung eines Ionenaustausches.

Eine weitere Verbesserung der erfindungsgemäßen Unterbindung einer Wasseraufnahme in Alginatmaterial wird erzielt, falls anstelle der herkömmlichen organischen Puffermoleküle in der Fällungslösung als Puffersubstanz Histidin oder eine andere biologische Puffersubstanz enthalten ist. Histidin besitzt einen isoelektrischen Punkt beim pH-Wert pH = 7.2. Ein bevorzugter Konzentrationsbereich für den Histidin-Zusatz beträgt rd. 1 bis 10 mM.

Das gewünschte Anschwellen des vernetzten Alginatmaterials wird erfindungsgemäß durch weitere Zusätze in der Alginatlösung und/oder Fällungslösung verringert. Dies ist in den Kurven (c) und (d) der Fig. 1 illustriert. So kann eine dauerhafte Stabilisierung der Alginat-Beads erzielt werden, wenn der Alginatlösung und/oder der Fällungslösung Proteine zugesetzt werden, die dazu eingerichtet sind, eine Dehydration der Alginate während der Polymerisation (Vernetzung) zu verhindern.

Beispiele für derartige Protine sind menschliche oder tierische Seren (z.B. fetales Kälberserum (FCS), menschliches Albumin-Serum oder Proteine aus dem Blut des jeweiligen Patienten). Bevorzugte Proteinkonzentrationen liegen im Bereich von 4 bis 20%. Ein besonders stabiles Alginatmaterial wurde mit einer 10%-igen Proteinkonzentration in der 1%-igen Alginatlösung und in der Fällungslösung erzielt. Bei einer 30-minütigen Inkubation in einer 6 mM-Na₂SO₄-Lösung ergibt sich der in Fig. 1 mit gefüllten Quadraten bzw. Dreiecken gezeichnete Kurvenverlauf (c) bzw. (d).

Für klinische Anwendungen, d.h. für Transplantatbehandlungen von Patienten, wird die Konzentration der Serumproteine vorzugsweise so ausgewählt, daß eine Anpassung des osmotischen Druckes in der jeweiligen Lösung an den osmotischen Druck der Proteine im Blut erzielt wird. Die Proteinkonzentration wird entsprechend der vorab zu ermittenden Proteinkonzentration im Patientenblut gewählt. Es müssen mit dem jeweiligen Patienten biokompatible Proteine (z.B. körpereigene Proteine) verwendet werden.

Alternativ können anstelle des fetalen Kälberserums auch Knochenpulver (zermörserte Knochen) oder Implantationsstoffe, wie z.B. Cementex (Synthetic Bone Source) verwendet werden.

Zur Verbesserung der Matrixstabilität ist es ergänzend zum Proteinzusatz oder auch alternativ zu diesem möglich, der Alginatlösung und/oder der Fällungslösung biokompatible, hydrophobe Substanzen oder Emulsionen zuzusetzen, die dazu eingerichtet sind, Zwischenräume zwischen den Ketten der Alginatmoleküle während des Vernetzungsvorganges zu füllen, wodurch der für die wässrige Lösung verfügbare Platz verringert wird. Beispiele für derartige Substanzen sind Perfluorkohlenwasserstoffe. Perfluorkohlenwasserstoffe besitzen den Vorteil, daß sie sich bereits bei medizinischen Anwendungen bewährt haben und daß sie sich als Markierungssubstanzen für NMR-Untersuchungen eignen. So ist es beispielsweise möglich, den Sauerstoffpartialdruck in einem Implantat durch NMR-Messung des 19 F-Signnals von Perfluorkohlenwasserstoffen nicht-invasiv zu messen und damit eine Aussage über die Lebensbedingungen des Implantats zu gewinnen. Ein Beispiel für eine verwendete hydrophobe Substanz ist Fluorosol. Falls dieses im Verhältnis 1:1 vor der Vernetzung der Alginatlösung und der Fällungslösung zugesetzt wird, so ergibt sich ebenfalls eine Dauerstabilität wie bei den Kurvenverläufen (c) und (d).

Die Kurvenbilder (b) bis (d) zeigen im Vergleich zu dem Kurvenbild (a) eine erheblich verlängerte Lebensdauer der Alginatkapseln. Dies ermöglicht die Durchführung von Transplantationsuntersuchungen an tierischen oder menschlichen Organismen im Rahmen der interessierenden Zeitbereiche. Die Transplantation erfindungsgemäß hergestellter Alginat-Beads in Muskelgewebe und unter die Nierenkapsel von sogenannten Lewis-Ratten hat eine hervorragender Langzeitstabilität ergeben. So konnte mit fluorosolbehandelten Alginatkapseln eine Stabilität für über 2 Jahre nachgewiesen werden.

Weitere Vorteile für die Kapselstabilität werden erzielt, wenn bei der Alginatvernetzung simultan zum Einbau der Proteine und/oder des Fluorosols Gewebe oder Zellen der Nebenschilddrüsen imobilisiert werden.

## Patentansprüche

1. Verfahren zur Herstellung von Alginatmaterial, mit den Schritten:
- Bildung eines vernetzten Alginatmaterials aus einer Alginatlösung, und
- Behandlung des vernetzten Alginatmaterials mit einer Behandlungslösung, die zwei- oder mehrwertige Anionen enthält, die für eine Verbindung mit Überschuß-Kationen im vernetzten Alginatmaterial vorgesehen sind.

2. Verfahren gemäß Anspruch 1, bei dem die Behandlungslösung eine Sulfatlösung ist.

3. Verfahren gemäß Anspruch 2, bei dem die Behandlungslösung eine Na₂SO₄-Lösung ist.

4. Verfahren gemäß einem der vorhergehenden Ansprüche, bei dem die Bildung des vernetzten Alginatmaterials unter Anwesenheit biologischer Puffermoleküle in der Alginat- und/oder Fällungslösung erfolgt.

5. Verfahren gemäß Anspruch 4, bei dem als biologischer Puffer Histidin verwendet wird.

6. Verfahren gemäß einem der vorhergehenden Ansprüche, bei dem die Bildung des vernetzten Alginatmaterials unter Zusatz von Proteinen in der Alginat- und/oder Fällungslösung erfolgt, die dazu eingerichtet sind, eine Dehydration des Alginatmaterials während der Vernetzung zu verhindern.

7. Verfahren gemäß Anspruch 6, bei dem die Proteine menschliche oder tierische Serumproteine umfassen.

8. Verfahren gemäß Anspruch 7, bei dem die Proteine fetales Kälberserum, menschliches Albumin-Serum, Proteine aus dem Blut eines behandelten Patienten, Knochenpulver oder Implantationsstoffe umfassen.

9. Verfahren gemäß einem der vorhergehenden Ansprüche, bei dem die Bildung des vernetzten Alginatmaterials unter Anwesenheit einer biokompatiblen, hydrophoben Substanz oder Emulsion erfolgt, die dazu eingerichtet ist, Zwischenräume zwischen den Alginatketten während der Vernetzung zu füllen.

10. Verfahren gemäß Anspruch 9, bei dem die hydrophobe Substanz ein Perfluorkohlenstoff ist.

11. Verfahren gemäß einem der vorhergenden Ansprüche, bei dem die Behandlung des vernetzten Alginatmaterials in einer Na₂SO₄-Lösung mit einer Konzentration im Bereich von 0.4 bis 20 mM für eine Dauer von rd. 10 bis 60 Minuten erfolgt.

12. Transplantatkomposit, bestehend aus einem Alginatmaterial, das mit einem Verfahren gemäß einem der Ansprüche 1 bis 11 hergestellt ist, und einer Wirksubstanz, die aus biologischen Zellen, Gewebe und/oder Wirkstoffen besteht.

13. Transplantatkomposit gemäß Anspruch 11, bei dem das Alginatmaterial die Wirksubstanz kapselförmig umgibt.

## Claims

1. Method for producing alginate material by the steps:
- formation of a cross-linked alginate material from an alginate solution, and
- processing of the cross-linked alginate material with a processing solution containing divalent or multivalent anions intended to link with surplus cations in the cross-linked alginate material.

2. Method according to claim 1 in which the processing solution is a sulfate solution.

3. Method according to claim 2 in which the processing solution is a Na₂SO₄ solution.

4. Method according to any of the preceding claims in which formation of the cross-linked alginate material is produced in the presence of biological buffer molecules in the alginate and/or precipitating solution.

5. Method according to claim 4 in which histidine is used as a biological buffer.

6. Method according to any of the preceding claims in which formation of the cross-linked alginate material is produced by adding proteins to the alginate and/or precipitating solution, these being intended to prevent dehydration of the alginate material during cross linkage.

7. Method according to claim 6 in which the proteins comprise human or animal serum proteins.

8. Method according to claim 7 in which the proteins comprise fetal calf serum, human albumin serum, proteins from the blood of a treated patient, bone powder or implant substances.

9. Method according to any of the preceding claims in which formation of the cross-linked alginate material is produced in the presence of a biocompatible, hydrophobic substance or emulsion, this being intended to fill spaces between the alginate chains during cross linkage.

10. Method according to claim 9 in which the hydrophobic substance is a perfluoro hydrocarbon.

11. Method according to any of the preceding claims in which the cross-linked alginate material is processed in a Na₂SO₄ solution with a concentration between 0.4 and 20 mM for approx. 10 to 60 min.

12. Composite transplant consisting of an alginate material produced by a method according to anyone of claims 1 to 11, and an effective substance consisting of biological cells, tissue and/or effective components.

13. Composite transplant according to claim 11 in which the alginate material encapsulates the effective substance.

## Revendications

1. Procédé de fabrication d'une matière alginate, comprenant les étapes suivantes :
- formation d'une matière alginate réticulée, à partir d'une solution d'alginate et
- traitement de la matière alginate réticulée, à l'aide d'une solution de traitement contenant des anions bivalents ou polyvalents, lesquels sont prévus pour un composé présentant des cations excédentaires dans la matière alginate réticulée.

2. Procédé selon la revendication 1, dans lequel la solution de traitement est une solution de sulfate.

3. Procédé selon la revendication 2, dans lequel la solution de traitement est une solution de Na₂SO₄.

4. Procédé selon l'une quelconque des revendications précédentes, dans lequel la formation de la matière alginate réticulée a lieu en présence de molécules tampons biologiques dans la solution d'alginate et/ou dans une solution de précipitation.

5. Procédé selon la revendication 4, dans lequel le tampon biologique utilisé est de l'histidine.

6. Procédé selon l'une quelconque des revendications précédentes, dans lequel la formation de la matière alginate réticulée a lieu sous adjonction dans la solution d'alginate et/ou dans la solution de précipitation de protéines, qui sont réglées pour éviter une déshydratation de la matière alginate, au cours de la réticulation.

7. Procédé selon la revendication 6, dans lequel les protéines comprennent des protéines de sérum humain ou animal.

8. Procédé selon la revendication 7, dans lequel les protéines comprennent du sérum foetal de veau, du sérum d'albumine humain, des protéines issues du sang d'un patient traité, de la poudre d'os ou des substances d'implantation.

9. Procédé selon l'une quelconque des revendications précédentes, dans lequel la formation de la matière alginate réticulée a lieu en présence d'une substance ou d'une émulsion hydrophobe, biocompatible, laquelle est réglée pour remplir des intervalles entre les chaînes d'alginate, au cours de la réticulation.

10. Procédé selon la revendication 9, dans lequel la substance hydrophobe est un carbone perfluoré.

11. Procédé selon l'une quelconque des revendications précédentes, dans lequel le traitement de la matière alginate réticulée a lieu dans une solution de Na₂SO₄, présentant une concentration située dans la fourchette de 0,4 à 20 mM, pour une durée d'environ 10 à 60 minutes.

12. Composite de transplantation, constitué d'une matière alginate, laquelle est fabriquée par application d'un procédé selon l'une quelconque des revendications 1 à 11 et d'une substance active, constituée de cellules, de tissus et/ou de principes actifs biologiques.

13. Composite de transplantation selon la revendication 11, dans lequel la matière alginate enrobe la substance active, en forme de capsule.
